# DEMANDE DE BREVET EUROPEEN

(11) **EP 1 452 187 A1**
(43) Date de publication de la demande: **01.09.2004**
(21) Numéro de dépôt: 03004499.4
(22) Date de dépôt: 28.02.2003
(51) Int. Cl.: A61L 2/26, A47L 15/50, A61L 2/00, A61B 19/00, A61L 2/025, A61L 2/07, A61L 2/18

(54) **Panier pour le nettoyage, la décontamination ou la stérilisation de pièces ou instruments; procédé de fabrication et utilisation d'un tel panier**

(71) Demandeur: UMC S.A., 2520 La Neuveville (CH)
(72) Inventeur: Vollenweider, Florian, 2063 Saules (CH)
(74) Mandataire: North, Mathieu

(57) **Abrégé**

Ce procédé de fabrication d'un panier destiné au nettoyage, à la décontamination ou à la stérilisation de pièces ou d'instruments, notamment médicaux, utilise le perçage des parois (1) du panier sans utilisation de mèches, mais au moyen de laser, d'électroérosion, de poinçonnage, de grignotage ou de jet de liquide abrasif. Les parois sont reliées entre elles par des barres (2) qui sont fichées dans les trous pratiqués dans les parois. Les extrémités des barres sont ensuite soudées électriquement. Les parois aussi bien que les barres sont faites d'un même matériau, de préférence d'acier inoxydable ou de titane. L'absence de brasure évite des réactions chimiques imprévisibles. Le panier résultant du procédé est utilisable pour le traitement de pièces sortant de fabrication ou pour des instruments médicaux, notamment de chirurgie. Il est utilisable dans des processus de traitement à la vapeur, par liquide ou par ultrasons.

## Description

La présente invention se rapporte au domaine des paniers destinés à contenir des pièces ou instruments dans des machines ou dispositifs de nettoyage, de décontamination ou de stérilisation.

Les paniers habituellement utilisés actuellement sont constitués de treillis métalliques fixés sur une armature également métallique faite de barres, les barres étant soudées entre elles et le treillis lui-même soudé sur cette armature. La forme du panier est classique, c'est-à-dire parallélépipédique et rectangulaire, et les pièces ou instruments à nettoyer, à décontaminer ou à stériliser sont placés à l'intérieur du panier en vrac, sans moyens de les maintenir en place de façon à leur éviter de bouger et par conséquent de s'entrechoquer, par exemple sous la pression de jets de liquides nettoyants ou désinfectants, ou lors de transports. De tels chocs peuvent être très dommageables pour des pièces ou instruments de précision, tels que les instruments chirurgicaux.

Ce problème a fait l'objet de quelques solutions. Par exemple, le brevet US 4,577,755 propose un panier présentant notamment des fentes verticales dans lesquelles peuvent s'insérer les anneaux de ciseaux chirurgicaux, ainsi que des barres amovibles flexibles pour soutenir verticalement les branches des ciseaux. De la sorte, les ciseaux sont maintenus aussi bien verticalement qu'horizontalement. La construction, sans doute ingénieuse, est toutefois complexe et onéreuse.

Un autre exemple est fourni par le brevet JP 11,019,196, qui présente une série de supports formés de barres coudées sur lesquelles viennent s'accrocher les anneaux destinés à la préhension par l'index et le pouce pour tenir les ciseaux. Ceux-ci sont maintenus en place par leur poids, en porte-à-faux et sont maintenus latéralement par des décrochements verticaux que présentent les supports. D'autres formes de supports permettent de maintenir des pièces de configurations différentes. La difficulté dans ce genre de réalisations consiste dans la nécessité de former les supports, ce qui exige plusieurs opérations et renchérit la fabrication.

Un autre dispositif encore est proposé dans le brevet allemand DE 2929227, qui présente une grande complexité de construction, et qui permet, grâce à une série de poussoirs verticaux soumis à la pression de ressorts hélicoïdaux, de maintenir des instruments chirurgicaux allongés dans leur position. Les poussoirs, qui s'appuient chacun sur un point différent de l'instrument, s'avancent ainsi plus ou moins en fonction de l'épaisseur de l'instrument au point de contact. La complexité de sa construction rend toutefois prohibitif le prix de ce dispositif.

Le but de la présente invention est de proposer un procédé de construction simple, et par conséquent peu onéreux, d'un panier destiné au nettoyage, à la décontamination ou à la stérilisation de pièces ou d'instruments; un autre but de la présente invention est de fournir un panier résultant d'un tel procédé, ainsi que l'utilisation d'un tel panier dans des procédés de nettoyage, de décontamination ou de stérilisation particuliers, ou pour des pièces ou instruments déterminés.

Le procédé de fabrication objet de l'invention a trait à un panier qui comprend au moins deux plaques 1 jointes l'une à l'autre par plusieurs barres 2, lesdites plaques étant percées de trous 3 sans utilisation de mèches de perçage, lesdits trous 3 ayant un diamètre correspondant à celui desdites barres, les extrémités de chaque barre étant introduites chacune dans un trou d'une plaque et lesdites extrémités étant soudées dans lesdits trous.

En principe, les plaques sont placées verticalement, et les barres horizontalement. D'autres positions sont envisageables, selon les nécessités résultant des formes des pièces à traiter ou des machines dans lesquelles les paniers sont utilisés.

Dans une première forme d'exécution particulière de l'invention, la soudure des extrémités des barres (2) dans les trous (3) des plaques (1) est électrique.

Dans une deuxième forme d'exécution, qui peut s'appliquer à la précédente, les plaques (1) et les barres (2) sont en acier inoxydable.

Dans une troisième forme d'exécution, qui peut s'appliquer à la première, les plaques (1) et les barres (2) sont en titane.

Dans une quatrième forme d'exécution particulière de l'invention, qui peut s'appliquer aux précédentes, que les trous (3) sont percés par électro-érosion.

Dans une cinquième forme d'exécution, qui peut inclure les trois premières, les trous (3) sont percés par laser.

Dans une sixième forme d'exécution, qui peut inclure les trois premières formes d'exécution, les trous (3) sont percés par grignotage.

Dans une septième forme d'exécution, applicable aux trois premières, les trous (3) sont percés par poinçonnage.

Dans une huitième forme d'exécution, qui peut s'appliquer aux trois premières, les trous (3) sont percés par jet de liquide abrasif.

Dans une neuvième forme d'exécution, qui peut s'appliquer à toutes les forme d'exécution précédentes, le panier fabriqué par un procédé selon l'invention comprend au moins quatre plaques 1 reliées deux à deux par des barres, les plaques formant un quadrilatère.

Dans une dixième forme d'exécution, applicable aux neuf premières, certaines barres sont placées dans le panier de manière à maintenir les pièces ou instruments latéralement et d'autres barres de manière à maintenir ceux-ci verticalement.

Dans une première forme d'utilisation, applicable à l'une ou l'autre des formes d'exécution susmentionnées, le panier fabriqué selon l'invention est utilisé dans un nettoyage ou une stérilisation par vapeur.

Dans une deuxième forme d'exécution, applicable à la première forme d'utilisation, le panier fabriqué par un procédé selon l'invention est utilisé dans un nettoyage ou une stérilisation par liquide

Dans une troisième forme d'utilisation, applicable aux deux premières, le panier fabriqué par un procédé selon l'invention est utilisé dans un nettoyage ou une stérilisation par ultrasons.

Dans une quatrième forme d'utilisation, qui est également applicable aux trois premières, le panier fabriqué par un procédé selon l'invention est utilisé pour le nettoyage, la décontamination ou la stérilisation d'instruments médicaux..

Dans une cinquième forme d'utilisation, le panier fabriqué par un procédé selon l'invention est utilisé pour le nettoyage, la décontamination ou la stérilisation de pièces sortant de fabrication.

Les dessins montrent, à titre d'exemple, un panier selon l'invention.

La figure 1 est une vue de dessus d'un panier selon l'invention.

La figure 2 est une coupe longitudinale d'un panier tel que montré à la figure 1.

La figure 3 est une coupe transversale d'un panier tel que montré à la figure 1.

La figure 4 est une vue partielle en coupe d'une plaque 1 percée de deux trous 3, une barre 2 étant fichée dans le trou inférieur, à fleur de la surface extérieure de la plaque 1.

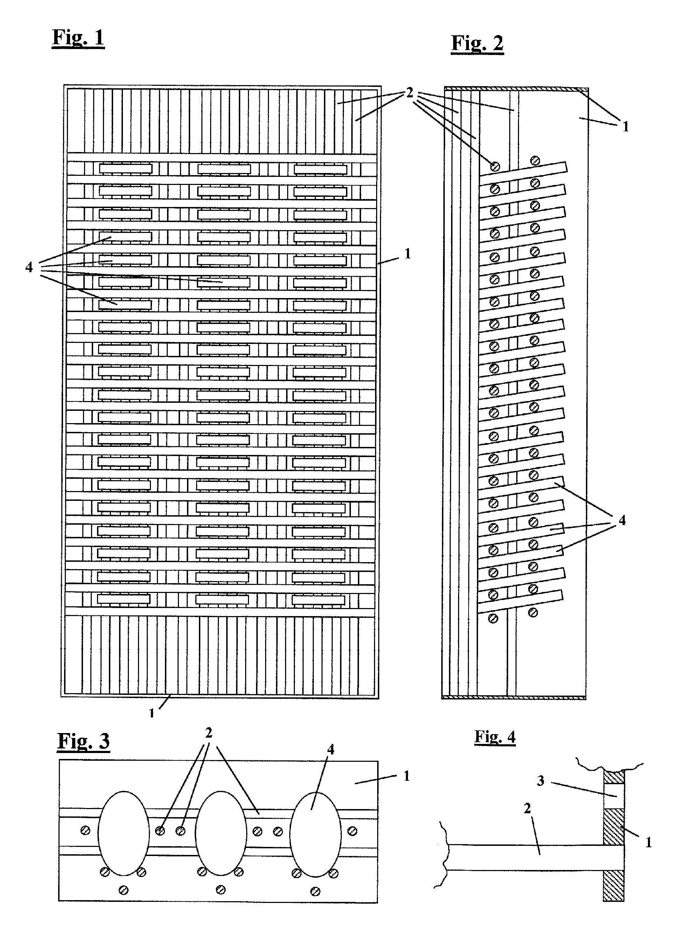

Dans la forme d'exécution préférée montrée ici dans les figures 1 à 4, les plaques 1 sont au nombre de quatre et forment une paroi continue sur un plan rectangulaire. Les barres 2 joignent entre elles les parois opposées. Comme le montre la figure 4, les parois sont percées de trous 3 dans lesquels viennent se ficher les barres, de façon que leur extrémité affleure à l'extérieur de la paroi. Les trous 3 ne sont pas percés au moyen de mèches. Ils le sont de préférence par laser. Selon les besoins et les quantités visées, le perçage peut aussi se faire, comme indiqué plus haut, par électroérosion, par poinçonnage, par grignotage, ou par jet de liquide abrasif. L'avantage de percements par de tels moyens évite des frais considérables, en évitant notamment le remplacement des mèches et la nécessité d'ébavurer les bords des trous. Une fois les extrémités des barres fichées dans leurs trous respectifs, on procède au soudage électrique. La soudure électrique évite la nécessité de recourir à une brasure, de sorte que le panier peut être constitué dans sa totalité en un seul matériau. Cette unité est précieuse lorsqu'il s'agit de nettoyer des pièces ou instruments sensibles, et d'éviter des réactions chimiques inattendues entre les liquides de nettoyage, de décontamination ou de stérilisation, les matières contenues dans la brasure et les matériaux dont sont constitués les pièces ou instruments traités.

Le panier représenté dans les dessins présente des barres longitudinales qui soutiennent verticalement les pièces ou instruments à traiter et qui sont placées dans le bas du panier, sous les pièces 4 qui remplissent le panier. D'autres barres longitudinales placées plus haut permettent de maintenir les pièces latéralement. De même, des barres transversales sont placées entre les deux parois les plus longues, de façon à maintenir longitudinalement les pièces ou instruments à traiter. Les pièces 4 à traiter représentées dans les dessins sont de simples plaques ovales.

Bien entendu, selon la forme des pièces ou instruments à traiter, le panier peut être structuré différemment : les barres peuvent être plus ou moins espacées et les parois prendre des formes différentes.

Les plaques 1 peuvent aussi présenter des échancrures ou découpures, que ce soit pour permettre aux fluides traitants de mieux circuler ou en vue d'un allègement.

Il peut aussi être envisagé de faire un panier présentant seulement deux plaques 1, pour des applications particulières.

De préférence, le panier est constitué de plaques et barres en acier inoxydable. Un autre matériau utilisable et présentant des avantages est le titane.

L'invention est utilisable notamment dans le nettoyage, la décontamination et la stérilisation d'instruments médicaux, plus particulièrement chirurgicaux. Le panier peut aussi être utilisé dans le nettoyage de pièces sortant de fabrication.

Le panier peut être utilisé dans toutes sortes de procédés de nettoyage, de décontamination ou de stérilisation, que l'on utilise de la vapeur, un liquide ou des ultrasons.

## Revendications

1. Procédé de fabrication d'un panier pour le nettoyage, la décontamination ou la stérilisation de pièces ou instruments (4), ledit panier comprenant au moins deux plaques (1) jointes l'une à l'autre par plusieurs barres (2) , **caractérisé en ce que** lesdites plaques sont percées de trous (3) sans utilisation de mèches de perçage, lesdits trous ayant un diamètre correspondant à celui desdites barres, **en ce que** les extrémités de chaque barre sont introduites chacune dans un trou d'une plaque, et **en ce que** lesdites extrémités sont soudées dans lesdits trous.

2. Procédé selon la revendication 1, **caractérisé en ce que** la soudure des extrémités des barres (2) dans les trous (3) des plaques (1) est électrique.

3. Procédé selon l'une des revendications 1 ou 2, **caractérisé en ce que** les plaques (1) et les barres (2) sont en acier inoxydable.

4. Procédé selon l'une des revendications 1 ou 2, **caractérisé en ce que** les plaques (1) et les barres (2) sont en titane.

5. Procédé selon l'une des revendications 1 à 4, **caractérisé en ce que** les trous (3) sont percés par électro-érosion.

6. Procédé selon l'une des revendications 1 à 4, **caractérisé en ce que** les trous (3) sont percés par laser.

7. Procédé selon l'une des revendications 1 à 4, **caractérisé en ce que** les trous (3) sont percés par grignotage.

8. Procédé selon l'une des revendications 1 à 4, **caractérisé en ce que** les trous (3) sont percés par poinçonnage.

9. Procédé selon l'une des revendications 1 à 4, **caractérisé en ce que** les trous (3) sont percés par jet de liquide abrasif.

10. Panier fabriqué par un procédé selon l'une des revendications 1 à 9 **caractérisé en ce qu'**il comprend au moins quatre plaques (1) reliées deux à deux par des barres, les plaques formant un quadrilatère.

11. Panier selon la revendication 10, **caractérisé en ce que** certaines barres sont placées de manière à maintenir les pièces ou instruments latéralement et d'autres barres de manière à maintenir ceux-ci verticalement.

12. Utilisation d'un panier fabriqué par un procédé selon l'une des revendications 1 à 9, ou présentant les caractéristiques de l'une des revendications 10 ou 11, ou de ces deux revendications, dans un nettoyage ou une stérilisation par vapeur.

13. Utilisation d'un panier fabriqué par un procédé selon l'une des revendications 1 à 9, présentant les caractéristiques de l'une des revendications 10 ou 11, ou de ces deux revendications, dans un nettoyage ou une stérilisation par liquide.

14. Utilisation d'un panier fabriqué par un procédé selon l'une des revendications 1 à 9, ou présentant les caractéristiques de l'une des revendications 10 ou 11, ou de ces deux revendications, dans un nettoyage ou une stérilisation par ultrasons.

15. Utilisation d'un panier selon l'une des revendications 12 à 14 pour le nettoyage, la décontamination ou la stérilisation d'instruments médicaux.

16. Utilisation d'un panier selon l'une des revendications 12 à 14 pour le nettoyage, la décontamination ou la stérilisation de pièces sortant de fabrication.
